# EUROPEAN PATENT APPLICATION

(11) **EP 4 494 466 A1**
(43) Date of publication of application: **22.01.2025**
(21) Application number: 23382745.0
(22) Date of filing: 20.07.2023
(51) Int. Cl.: A01N 35/02, A01N 41/12, A01N 43/18, A01P 3/00, A01P 7/02, A61K 31/11, A61K 31/382, A61P 31/10, A61P 33/14, C07C 323/22

(54) **SULFUR-CONTAINING COMPOUNDS AND USE THEREOF**

(71) Applicant: Consejo Superior de Investigaciones Científicas (CSIC), 28006 Madrid (ES); Universidad de Granada, 18071 Granada (ES); Coopaman S.C.L., 16660 Las Pedroñeras Cuenca (ES)
(72) Inventor: GONZALEZ COLOMA, Ana Azucena, Madrid (ES); ANDRES YEVES, Maria Fe, Madrid (ES); DIAZ HERNANDEZ, Carmen Elisa, San Cristóbal de La Laguna (ES); FERNANDEZ BARRERO, Alejandro, Granada (ES); QUILEZ DEL MORAL, Jose F., Granada (ES)
(74) Representative: Pons

(57) **Abstract**

The present invention relates to the use of sulfur-containing compounds as biocides, preferably, as antifungal and/or ixodicidal agents. The present invention also relates to a method to control phytopathogens or pest organisms that affect plants and animals by treating them with said sulfur-containing compounds. Additionally, biocides compositions and specific sulfur-containing compounds are described.

## Description

The present invention relates to sulfur-containing compounds with biocidal activity and their use to treat plants and/or animal that are affected by pathogens, pest organisms or ectoparasites.

Therefore, the present invention belongs to the field of agriculture and veterinary science, and, more specifically, to the biocides sector.

### STATE OF ART

As consequence of the known risk and impact of synthetic pesticides on environment and human health (Aktar, M.W. et al. Impact of pesticides use in agriculture: their benefits and hazards. Interdiscip. Toxicol. 2009, 2, 1-12; Rani, L. et al. An extensive review on the consequences of chemical pesticides on human health and environment. J. Clean. Prod. 2021, 283, 124657; Kumar, R. et al. impact of pesticide toxicity in aquatic environment. Biointerface Res. Appl. Chem. 2021, 11, 10131-10140) severe restrictions on the use of these chemical substances are being proposed around the world. All this is causing an increasing interest in developing new pesticides derived from natural products (Marrone, P.G. Pesticidal natural products - status and future potential. Pest Manag. Sci. 2019, 75, 2325-2340).

Regarding pesticides derived from natural products, an example is found in document WO2002/017937A1, which refers to aqueous or organic extracts obtained from the fermentation of *Guignardia sp.* fungi isolated from the leaves of *the Spondias mombin* tree; said extracts are effective against bacteria, yeasts and fungi.

The nematicide activity of garlic hydrolate byproduct (Galisteo, A. et al. Valorization of the hydrolate byproduct from the industrial extraction of purple Allium sativum essential oil as a source of nematicidal products. Life. 2022, 12, 905) has been recently described. It should be noted that the world production of garlic in 2020 was about 28 million metric tons. Therefore, the use of garlic oil or extracts adds value to this residue of the essential oil industry.

On the other hand, many animals have parasites that live on the surface of their bodies, such as lice, fleas and ticks. These parasites bite the animal's skin and ingest its blood, often injecting foreign proteins with, anticoagulant, properties. This can irritate the animal, initiate allergic, responses, and may cause infections. These parasites can have a negative effect on any people the animal comes into contact with, or environments that the animal frequents. This issue if of greater significance when the animal is-domesticated and lives in close contact with its owner, arid in particular in the case of ticks. Several solutions have been proposed to repel or eradicate parasites and conventional treatments, include the application of a parasiticidal pesticide to the animal.

Even though some biocides of natural origin have been disclosed in the state of the art, traditionally, the usual method for the control of phytopathogens and pest organisms affecting plants and animals has been the application of synthetic chemicals, which, in addition to their high economic cost, entail a serious environmental damage. However, new regulations have drastically limited the number of chemicals and phytosanitary products for the control of diseases caused by pathogens. A similar situation can be described for the control of animal ectoparasites such as ticks. For all these reasons, there is currently a clear need to search for new, effective and less toxic active compounds as alternatives to the known ones. With this aim, the present invention proposes compounds from natural origin to be used as biocides in agriculture and veterinary science.

### DESCRIPTION OF THE INVENTION

The inventors have found that some compounds isolated from a garlic extract have biocidal activity, more particularly, antifungal and/or ioxicidal activity (in the order of µg/ml), so that they can be used as biocides in agriculture and veterinary, in particular, against fungi and ticks.

Then, in a first aspect, the present invention relates to the use of a compound of formula 1, 2 or 3 (hereinafter, compounds of the invention), 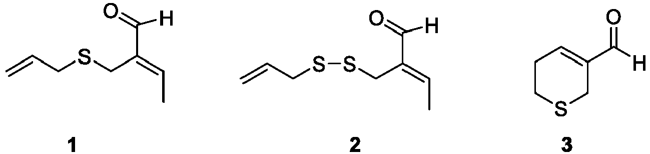 or a composition comprising the compound of formula 1, 2, 3 or any combination thereof, as a biocide to control phytopathogens or pest organisms that affect plants.

The term "control" that is used in the present specification means that the active compounds are effective for reducing the occurrence ratio of a phytopathogen or pest organism in a plant infected with it to an innoxious level. More specifically, the term "to control" means that the compounds of the present invention are effective for destroying parasites, inhibiting growth, or propagation thereof.

The term "phytopathogen" as used herein refers to a pathogenic organism that infects a plant.

In a preferred embodiment, the phytopathogens or pest organisms that affect plants are selected from ticks and fungi. Therefore, so thatthe compounds of the invention, or a composition comprising them, are preferably used as antifungal and/or ixodicidal (tick-killing ) agents. More preferably, the fungi are selected from *Aspergillus niger* and *Botrytis cinerea* and/or the tick species is *Hyalomma lusitanicum.*

In another aspect, the present invention relates to a method to control phytopathogens or pests that affect plants, wherein said method comprises treating the plant with an effective amount of a compound of the present invention (compound of formula 1, 2, or 3).

The term "effective amount" is used herein to describe that amount of a compound of the present invention necessary to achieve the desired level of inhibition or control of phytopathogens or pest organisms when applied to plants. The effective amount may optionally increase or decrease depending on the compound of the invention used, the type of formulation, the time, place and method of application, the type of plant pathogen or pest organism and the degree of damage desired.

In a preferred embodiment, the method refers to the control of fungi and/or ticks, more preferably, to control fungi selected from *Aspergillus niger* and *Botrytis cinerea* and/or the species of ticks *Hyalomma lusitanicum.*

In order to treat plants, the application of the compounds of the invention is preferably carried out by direct spraying onto the plant where said organisms are found, or on the substrate (soil) where the plant is located.

Another aspect of the invention relates to a compound of formula 1, 2 or 3: 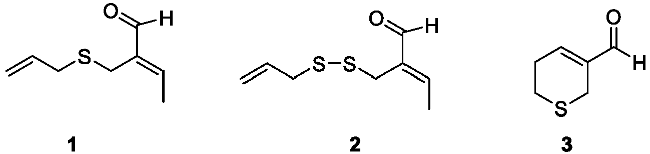 or a composition comprising the compound of formula 1, 2, 3 or any combination thereof, for use in the treatment of an animal infested with an ectoparasite.

The term "ectoparasite" corresponds to a parasite that lives permanently or occasionally on the surface or the skin of a host organism. Examples of ectoparasites are ticks and fungi.

The term "animal" in the present invention includes human mammals. Preferably the animal is selected from livestock and domestic mammals, such as rom cattle, sheep, goats, pigs, dogs, cats and horses.

In a preferred embodiment, the ectoparasite is selected form fungi and ticks. Then, the compounds of the invention, or a composition comprising them, are preferably used as as antifungal and/or ixodicidal (tick-killing) agents. More preferably, the fungi are selected from *Aspergillus niger* and *Botrytis cinerea* and/or the tick species is *Hyalomma lusitanicum.*

In a preferred embodiment, the compounds for use to treat animals are applied topically and, preferably, by direct spraying onto the animal where the ectoparasite is located.
In the present invention, the activity against fungi is determined by mycelial growth inhibition assays on a plate.

The ixodicidal activity is determined on larvae by cellulose contact toxicity.

Preferably, to be applied, the compounds of the invention are taking part of a biocidal composition.

Then, in another aspect, the present invention refers to a biocidal composition (composition with biocidal activity) comprising a compound of formula 1, 2, 3 or combinations thereof.

"Biocidal activity" means the ability to control at least one plant pathogen, or pest organism affecting plants and animals ectoparasites through different mechanisms of action. Such control includes the prevention of the action or direct destruction of such organisms harmful to public health, to an animal and to agriculture during production, storage, transport, distribution and processing of agricultural products and their derivatives. Examples of plant pathogens or pest organisms affecting plants and animals ectoparasites, which are included in the present invention are, without limitation, fungi and ticks.

The present invention also relates to the use of said biocidal composition to control phytopathogens or pest organisms that affect plants and to said biocidal composition for use to treat an animal infested with an ectoparasite.

The biocidal composition may additionally comprise vehicles or agents (surfactants, auxiliary agent as fixing agent and dispersing agent, stabilizers, etc) that facilitate its conservation, handling and application.

The term "vehicle" denotes a compound or material with which the active compound is combined to facilitate its application. This vehicle is thus generally inert, and it must be agriculturally / veterinary acceptable (not harmful to plants and animals).

As the expert in the state of the art will know, in the application of phytosanitary / veterinary products, solid vehicles, liquid vehicles, gaseous vehicles, etc., and, if necessary, surfactants and auxiliary agents are used for formulation of phytosanitary compositions. For example, vehicles to formulate forms such as emulsifiable concentrates, wettable powders, flowable liquids, (e.g., suspension in water, emulsion in water, etc.), powders, aerosols and the like can be added, so that different forms of the composition of the invention, such as those mentioned, are possible.

Examples of solid vehicles are fine powders or granules of clays (e.g., kaolin clay, diatomaceous earth, synthetic hydrated silicon oxide, bentonite, Fubasami clay, acid clay, etc.), talcs, ceramics and other inorganic minerals (e.g., sericite, quartz, sulfur, activated carbon, calcium carbonate, calcium silicate, silica, etc.), commercial fertilizers (e.g., ammonium sulfate, ammonium phosphate, ammonium phosphate, etc.) and the like.

Examples of liquid vehicles are water, alcohols (e.g., methanol, ethanol, etc.), ketones (e.g., acetone, methyl ethyl ketone, etc.), aromatic hydrocarbons (e.g., benzene, toluene, xylene, ethylbenzene, methylnaphthalene, etc), aliphatic hydrocarbons (e.g. hexane, cyclohexane, kerosine, gas oil, etc.), esters (e.g. ethyl acetate, butyl acetate, etc.), nitriles (e.g., acetonitrile, isobutyronitrile, etc.), ethers (e.g., diisopropyl ether, dioxane, etc.), acid amides (e.g., N,N-dimethylformamide, N,N- imethylacetamide, etc.), halogenated hydrocarbons (e.g., dichloroethane, trichloroethane, carbon tetrachloride, etc.), dimethyl sulfoxide, vegetable oils (e.g., soybean oil, cottonseed oil, etc.) and the like.

Examples of gaseous vehicles are spray agent, including flon gas, butane gas, LPG (liquefied petroleum gas), dimethyl ether, carbon dioxide gas and the like.

Examples of surfactants are alkyl sulfates, alkyl sulfonate salts, alkyl aryl sulfonates, alkyl aryl esters, polyoxyethylene compounds thereof, polyethylene glycol esters, polyhydroxyl alcohol esters, sugar alcohol derivatives and the like.

Examples of auxiliary agent, such as a fixing agent and dispersing agent, are casein, gelatin, polysaccharides (e.g., starch powder, gum arabic, cellulose derivative, alginic acid, etc.), lignin derivatives, bentonite, sugars, water-soluble polymers (e.g., synthetic polymers (e.g., polyvinyl alcohol, vinyl polypyrrolidone, acrylic polyacids, etc.) and the like.

Examples of stabilizers are isopropyl acid phosphate, BHT (2,6-di-di-tert-butyl-4-methylphenol), BHA (mixture of 2-tert-butyl-4-methoxyphenol and 3-tert-butyl-4-methoxyphenol), vegetable oils, mineral oils, surfactants, surface oils, mineral oils, surfactants, fatty acids or esters thereof and the like.

In another aspect, the present invention refers to a compound of formula 1 or 2.

Compounds of formula 1, 2 and 3 can be obtained from a natural source, in particular, from a hydrolate garlic extract, as detailed in the examples of the present invention.

Additionally, compound of formula 3 is commercially available (CAS No.: 30058-79-8).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Fig. 1****.** Gas chromatography-mass spectrometry (GC-MS) spectrum of fraction C obtained in the example.
**Fig. 2****.** Scheme showing a proposal for the generation of compounds 1 and 2.
**Fig. 3****.** GC-MS spectrum of fraction D obtained in the example (compound 3).
**Fig. 4****.** Scheme showing a proposal for the generation of compound 3 of the invention.

### EXAMPLES

Following, a protocol to obtain the compounds of the invention (compounds of formula 1, 2 and 3) from a garlic extract is described. Also, their ixodicidal and antifungal activity have been tested. Specifically, the compounds will be isolated form a "garlic hydrolate extract" referred as GHE.

### Experimental part

### Materials and Methods:

Silica gel SDS 60 (35-70 µm) was used for flash column chromatography and monitored by thin layer chromatography (TLC) carried out on 0.25 mm E. Merck silica gel plates (60F-254) using UV light as the visualizing agent and solutions of phosphomolybdic acid in ethanol. HPLC with UV and RID detection was used. Semi-preparative HPLC separation were carried out on a column (5 µm Silica, 9.4 X 250 mm) at a flow rate of 2.0 mL/min in an Agilent Series 1100 instrument. NMR spectra were performed with a Varian Direct Drive 600 (¹H 600 MHz/¹³C 150 MHz), Varian Direct Drive 500 (¹H 500 MHz/¹³C 125 MHz), Varian Direct Drive 400 (¹H 400 MHz/¹³C 100 MHz) and BRUKER Avance NEO (¹H 400MHz/¹³C 100 MHz) spectrometers. High-resolution MS were determined on an Autospec-Q VG-Analytical (FISONS) mass spectrometer. DEPT-135 and two-dimensional (COSY, HSQC, HMBC, NOESY) NMR spectroscopy was used where appropriate to assist the assignment of signals in the ¹H and ¹³C NMR spectra.

The volatile compounds of hydrolate fractions were analyzed by gas chromatography coupled to mass spectrometry (GC-MS) using a GC-2010 (Shimadzu, Kioto, Japan) equipment coupled to a GCMS-QP2010 (Shimadzu, Kioto, Japan) mass detector, equipped with a Simple Quadrupole analyzer, an automatic injector (AOC-20i) (Shimadzu, Kioto, Japan) and a (95%) dimethyl- (5%) diphenyl polysiloxane capillary column (30 µm × 0.25mm ID and 0.25 µm phase thickness) (Teknokroma TRB-5, Barcelona, Spain). The samples (in DCM) were detected by electronic impact at 70 e with Helium as a carrier gas. The working conditions were as follows: Split mode injection (1 µL injected), division ratio (20:1), injector temperature 300 ºC, transfer line temperature 250 ºC and ionization source temperature 220 ºC. The initial temperature was 70 ºC, heating up to 290 ºC at 6 ºC/min plus 20 min leaving at 290 ºC. Mass spectra and retention time are used to identify compounds by comparison with the Wiley and NIST17 databases (Wiley 275 Mass Spectra Database, 2001, Palmer, Massachusetts, USA; NIST Mass Spectra Database, 2017, Gaithersburg, Maryland, USA).

### Plant materials

The purple garlic plant is cultivated by Coopaman S.A. in Las Pedroñeras, Cuenca, Spain. At harvest, the undersize garlic bulbs (<36 mm) are considered garlic waste and were used for the preparation of the extracts.

### Extraction and Fractionation

The GHE was prepared as described previously by the inventors (Galisteo, A. et al. Valorization of the hydrolate byproduct from the industrial extraction of purple Allium sativum essential oil as a source of nematicidal products. Life. 2022, 12, 905).

Briefly, undersize garlic bulbs (equatorial diameter below 37 mm) were extracted by steam distillation in an industrial plant equipped with a vessel of 1080 L. Garlic essential oil was separated by decantation from the aqueous phase, hydrolate, with an oil yield of 0.2% (w/w).

A sample of garlic hydrolate was absorbed with activated carbon (2 L hydrolate/80 g of carbon) and stirred for 30 min at room temperature. The resulting activated carbon was separated from the liquid by filtration on a Büchner with a cellulose filter and dried at 45ºC for 24 h.

The solid part was extracted with AcOEt in a Soxhlet for 12 h. The solvent was evaporated under reduced pressure to give 8.0 g of organic fraction (hydrolate OF) with a yield of 0.032% (w/v). The GHE (8 g) was chromatographed in a Si column using mixture of solvents (hexane (H), methyl *terc*-butyl eter (MTBE) and ethyl acetate (EtOAc)) of increasing polarity to obtain seven fractions:
- Fraction **A** (H): It was constituted by a mixture of hydrocarbons and esters of fatty acids as deduced from its GC-MS analysis.
- Fraction **B** (H-EtOAc (9-1)): This fraction (5.5 g) was analyzed by GC-MS showing a high content of organosulfur compounds, with diallyl disulfide (DADS), diallyl trisulfide (DATS) and methyl allyl trisulfide (MATS) being the main components (These substances are also the main components of the essential oil of garlic: Kocić-Tanackov, S. et al. Effects of onion (Allium cepa L.) and garlic (Allium sativum L.) essential oils on the Aspergillus versicolor growth and Sterigmatocystin production. J. Food Sci. 2012, 77, 278-284).
- Fraction **C** (H-EtOAc (4-1)): This fraction consisted of 308 mg of a mixture of compounds **1** and **2,** as deduced from its GC-MS analysis. 20 mg of this mixture were subjected to semipreparative HPLC with an isocratic elution (H-MTBE, 9-1) in a flow of 4 mL/min to obtain 9 mg of **1** (Rt=7.84 min) and 3 mg of **2** (Rt=8.59 min). 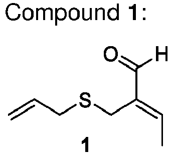 ¹H NMR (600 MHz, CDCl₃) δ 9.43 (s, 1H), 6.72 (q, *J* = 7.1 Hz, 1H), 5.86 (ddt, *J* = 17.0, 10.0, 7.0 Hz, 1H), 5.22 (dq, *J* = 17.0, 1.2 Hz, 1H), 5.15 (dq, *J* = 10.0, 1.2 Hz, 1H), 3.36 (s, 2H), 3.18 (dt, *J* = 7.0, 1.2 Hz, 2H), 2.09 (d, *J* = 7.1 Hz, 3H). ¹³C NMR (151 MHz, CDCl₃) δ 193.42, 151.44, 141.60, 134.18, 117.35, 35.69, 22.98, 15.25. HRMS TOF (ESI+) m/z calculated for C₈H₁₃OS [M + H]+ 157.0668, found 157.0687. 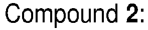 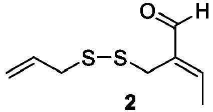 ¹H NMR (600 MHz, CDCl₃) δ 9.43 (s, 1H), 6.81 (q, *J* = 7.1 Hz, 1H), 5.88 (ddt, *J* = 17.0, 10.0, 7.4 Hz, 1H), 5.23 (dq, *J* = 17.0, 1.2 Hz, 1H), 5.19 (ddd, *J* = 10.0, 1.2, 0.9 Hz, 1H), 3.64 (s, 2H), 3.36 (dt, *J* = 7.4, 0.9 Hz, 2H), 2.14 (d, *J* = 7.1 Hz, 3H). ¹³C NMR (151 MHz, CDCl₃) δ 192.91, 152.15, 140.35, 133.17, 118.81, 41.91, 30.78, 15.62. HRMS TOF (ESI+) m/z calculated for C₈H₁₃OS₂ [M + H]+ 189.0399, found 189.0408.
- Fraction **D** (H-EtOAc (7-3)): The GC-MS analysis of this fraction (284 mg) showed a major unknown peak. 20 mg of fraction **D** was purified by semipreparative HPLC with an isocratic elution (H-MTBE, 8-2) in a flow of 4 mL/min to obtain 11 mg of **3** (Rt=10.31 min). 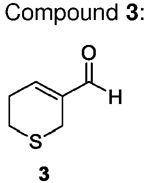 ¹H NMR (600 MHz, CDCl₃) δ 9.38 (s, 1H), 6.91 (tt, 1H), 3.33 (q, *J* = 2.2 Hz, 2H), 2.80 (t, *J* = 5.7 Hz, 2H), 2.72 - 2.65 (m, 2H). ¹³C NMR (126 MHz, CDCl₃) δ 192.78, 150.93, 138.99, 27.53, 24.72, 22.35. HRMS TOF (ESI+) m/z calculated para C₆H₉OS [M + H]+ 129.0359, found 129.0374.
- Fraction **E** (H-EtOAc (7-3)): This fraction (176 mg) was analyzed by GC-MS to show a high content of alcohols and fatty acids.
- Fraction **F** (H-EtOAc (3-2)): The GC-MS analysis of this fraction (463 mg) showed a major peak assigned to the compound **5:** 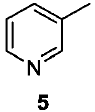
- Fraction **G** (H-EtOAc (1-1: The GC-MS analysis of this fraction (139 mg) showed a major peak assigned to compound **4.** 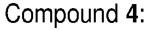 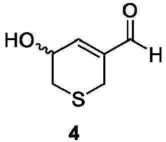 ¹H NMR (400 MHz, Acetone) δ 9.44 (s, 1H), 6.85 (dq, *J* = 2.6, 1.3 Hz, 1H), 4.59 (ddtd, *J* = 8.6, 5.3, 2.7, 2.0 Hz, 1H), 3.28 (dt, *J* = 17.6, 2.4 Hz, 1H), 3.06 (dq, *J* = 17.7, 1.7 Hz, 1H), 2.90 (ddt, *J* = 13.1, 5.3, 1.3 Hz, 1H), 2.65 (dd, *J* = 13.1, 8.6 Hz, 1H). ¹³C NMR (101 MHz, Acetone) δ 192.82, 152.67, 139.19, 65.16, 31.46, 21.36.

### Ixodicidal activity

*H. lusitanicum* engorged female ticks were collected in central Spain (Finca La Garganta, Ciudad Real) from their host (deer) and maintained at 22-24 °C and 70% RH until oviposition and egg hatching. Resulting larvae (4-6 weeks old) were used for the bioassays (Galisteo Pretel, A. *et al.* Germacrone derivatives as new insecticidal and acaricidal compounds: a structure-activity relationship. *Mol.* 2019, 24). Briefly, 50 µL of test solution were added to 25 mg of powdered cellulose at different concentrations (initial concentration of 20 mg/ml) and the solvent was evaporated. For each test, three replicates with 20 larvae each were used. Dead ticks were counted after 24 h of contact with the treated cellulose at the environmental conditions described, using a binocular magnifying glass. The larvicidal activity data are presented as percent mortality corrected according to Schneider-Orelli's formula (Schneider-Orelli, O. Entomologisches praktikum: Einführung in die land- und forstwirtschaftliche Insektenkunde; aus dem entomologischen institut der eidgenössischen technischen hochschule in Zürich; Sauerländer, 1947). Effective lethal doses (LC₅₀ and LC₉₀, the concentration that kills 50% or 90% of the organisms, respectively) were calculated by Probit Analysis (5 serial dilutions, STATGRAPHICS Centurion XVI, version 16.1.02, Statgraphics Technologies, Inc, P.O. Box 134, The Plains, Virginia 20198, USA).

### Antifungal activity

The fungal species *Aspergillus niger* and *Botrytis cinerea* came from the fungal collection of institute de Ciencias Agrarias-CSIC, Madrid, Spain where they are maintained. The antifungal activity of the hydrolate extract and fractions were determined using a modified spore germination inhibition growth assay (Sainz, P. et al. Chemical composition and biological activities of Artemisia pedemontana subsp. assoana essential oils and hydrolate. Biomol. 2019, 9). The hydrolate extract or fractions were dissolved in dimethyl sulfoxide (DMSO) at 1% and evaluated at the final concentrations indicated.

The spore suspensions were 7.5 × 10⁵ cells/mL in NaCl 0.9% for *A. niger* and 1 × 10⁷ cells/mL in distilled water for B. cinerea. Amphotericin B (5 µg/mL) was used as a positive control. The samples and spore suspensions (4 replicates) were placed on 96-well plates and incubated for 24 h (28 ºC for *A. niger* and 25 ºC for *B. cinerea*)*.* After the incubation process, 25 µL of an MTT (5 mg/mL) plus menadione (1 mM) solution in RMPIMOPS were added, the plates were incubated again for 3 h, the medium was removed, 200 µL of acidic isopropanol (95% isopropanol and 5% 1 M HCl) was added, and the plates were incubated for another 30 min. The absorbance was read at 490 nm in an Elisa reader. The IC₅₀ values (the effective dose to give 50% inhibition) were calculated by a regression curve of % spore germination inhibition on log dose.

### Results and Discussion

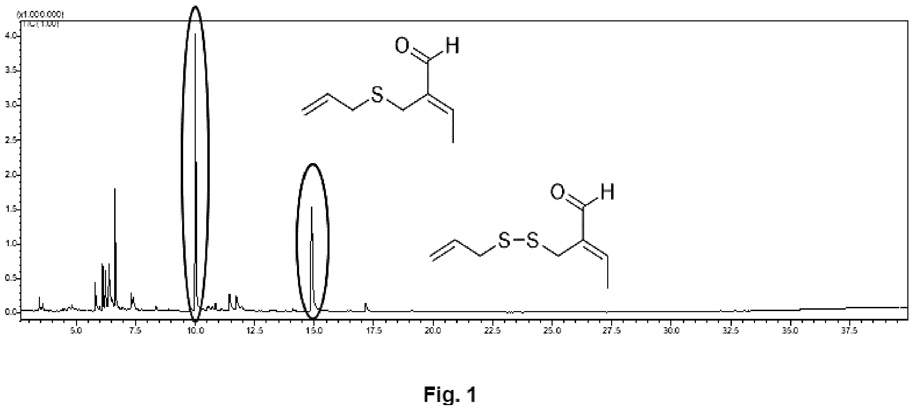

The study of the chemical composition of fraction **C** revealed, according to its GC-MS (Figure 1), the presence of two unknown major compounds.

This fraction was subjected to semipreparative HPLC, which allowed the isolation of compounds **1** and **2.** Compound **1** showed in its HRMS the molecular ion [M+H]+ at m/z 157.0687, corresponding to a molecular formula of C₈H₁₃OS. Analysis of its ¹H-NMR spectrum indicates the presence of a S-CH₂-CH=CH₂ moiety, as deduced from the coupling constants of the signals appearing at a δ 5,86, 5,22, 5,15 and 3,18 ppm (Table 1), data comparable to those exhibited by known compounds such as diallyl sulfide (DAS) (Herrera, C. ACS Appl. Mater. Interfaces 2019, 11, 35312-35318). The NMR signals at a δ 9,43 and 6.72 (¹H NMR) and 193.42 ppm (¹³C NMR) confirmed the presence of an α,β-unsaturated aldehyde. The multiplicity of this olefinic proton (q, *J* = 7.1 Hz) indicated the presence of a vicinal methyl, the latter appearing at 2.09 ppm (d, *J* = 7.1 Hz). Finally, the signal at δ 3,36 ppm could be attributed a methylene group bound to a un CH₂ bound to a sulfur atom. Assignment of the structure of 2-((allylthio)methyl)but-2-enal to 1 was confirmed on the basis of the long-range correlations observed in the HMBC experiment (Table 1).

**Table 1: NMR data of compound 1.**

| **Position** | **¹H (ppm)** | **¹³C (ppm)*** | **NOE** | **HMBC** |
|---|---|---|---|---|
| 1 | 9.43 s | 193.42 (CH) | H₆ | C₄, C₅, C₆ |
| 2 | - | 141.60 (C) | - | - |
| 3 | 6.72 *q* (*J* = 7.1 Hz) | 151.44 (CH) | H₇, H₈ | C₄, C₅, C₇ |
| 4 | 2.09 *d* (*J* = 7.1 Hz) | 15.25 (CH₃) | H₄, H₆ | C₄, C₅, C₆, |
| | | | | C₈ |
| 5 | 3.36 s | 22.98 (CH₂) | H₃, H₇ | C₃, C₅, C₆, C₈ |
| 6 | 3.18 *dt* (*J* = 7.0, 1.2 Hz) | 35.69 (CH₂) | - | C₁₅ C₂, C₄ |
| 7 | 5.86 ddt (*J* = 17.0, 10.0, 7.0 Hz) | 134.18 (CH) | - | C₃ |
| 8_{α} | 5.22 *dq* (*J* = 17.0, 1.2 Hz) | 117.35 (CH₂) | - | C₂, C₃ |
| 8_{β} | 5.15 *dq* (*J* = 10.0, 1.2 Hz) | 117.35 (CH₂) | - | C₃ |

| | | | | |
|---|---|---|---|---|
| ** HSQC-based assignment* | | | | |

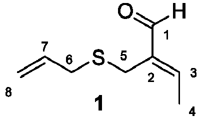

Finally, the geometry of the trisubstituted double bond was established after analysis of 1D NOE experiments. Thus, the observation of NOE at Me 4 after irradiation of H6 confirmed the *E* geometry of this unsaturation. This geometry is corroborated after noticing the γ-shielding effect experienced by C5.

Compound **2** was assigned the molecular formula C₈H₁₃OS₂ from its HRMS ([M+H]+, m/z 189. 0399). Its NMR spectra were very similar to those of **1**, with the main differences being the chemical shifts of the carbons bound to the sulfur atom. Thus, while these carbons resonate in compound **1** at δ 35,69 and 22,98 ppm (Table 1), the corresponding resonances appeared in compound **2** at δ 41,91 30,78 ppm (Table 2), which suggested the presence of a disulfide bridge in compound **2.** This proposal is supported after noticing that similar chemical shifts differences were found when comparing the NMR data of DAS with those of DADS or DATS (Herrera, C. et al. Polysulfides Synthesized from renewable garlic components and repurposed sulfur form environmentally friendly adhesives. ACS Appl. Mater. Interfaces 2019, 11, 35312-35318; Baker, A. et al. Flow synthesis of symmetrical Di- and trisulfides using phase-transfer catalysis. J. Flow Chem. 2013, 3, 118-121).

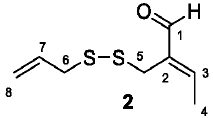

**Table 2: NMR data of compound 2.**

| **Position** | **¹H (ppm)** | **¹³C (ppm)*** | **NOE** | **HMBC** |
|---|---|---|---|---|
| 1 | 9.43 s | 192.91 (CH) | H₆ | C₄, C₅ |
| 2 | - | 140.35 (C) | - | - |
| 3 | 6.81 *q* (*J* = 7.1 Hz) | 152.15 (CH) | H₇, H₈ | C₄, C₇, C₈ |
| 4 | 2.14 *d* (*J* = 7.1 Hz) | 15.62 (CH₃) | H₄, H₆ | C₄, C₅, C₆, C₈ |
| 5 | 3.64 *s* | 30.78 (CH₂) | H₃, H₇ | C₅, C₆, C₈ |
| 6 | 3.36 *dt* (*J* = 7.4, 0.9 Hz) | 41.91 (CH₂) | - | C₁, C₂ |
| 7 | 5.88 ddt (*J* = 17.0, 10.0, 7.4 Hz) | 133.17 (CH) | - | C₃ |
| 8_{α} | 5.23 *dq* (*J* = 17.0, 1.2 Hz) | 118.81 (CH₂) | - | C₂, C₃ |
| 8_{β} | 5.19 *ddd* (*J* = 10.0, 1.2, 0.9 Hz) | 118.81 (CH₂) | - | C₃ |

| | | | | |
|---|---|---|---|---|
| **HSOC-based assignment.* | | | | |

The generation of compounds **1** and **2** can be rationalized considering the aldol condensation between synthons **I** and **II** and a molecule of acetaldehyde (**III**) (Scheme 1). Synthons **I** and **II** would be, in turn, originated via Michael addition of allyl mercaptan (**IV**) or 2-propenepethiol (**V**) to acrolein (**VI**), respectively. The fact that compounds **III-VI** are known to be found in garlic extracts (Vollrath, R.E. et al. Bactericidal properties of acrolein. Proc. Soc. Exp. Biol. Med. 1937, 36, 55-58; Yu, T.H. et. al. Volatile compounds generated from thermal degradation of alliin and deoxyalliin in an aqueous solution. J. Agric. Food Chem. 1994, 42, 146) supported this proposal.

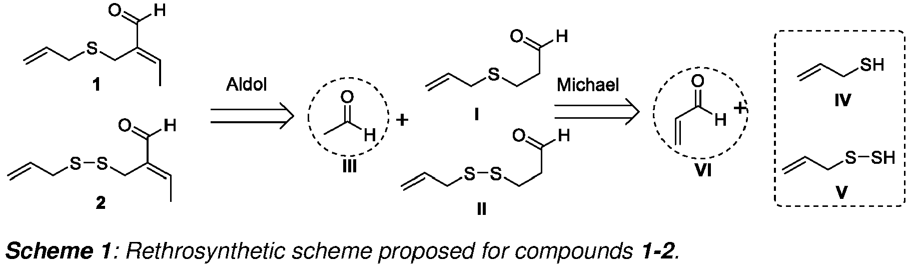

The origin of synthons **IV** and **V** may lay in the aqueous degradation of allicin (**VII**) or to the reduction of polysulfides present in garlic (Münchberg, U. et al. Polysulfides as biologically active ingredients of garlic. Org. Biomol. Chem. 2007, 5, 1505-1518; Block, E. et al. J. Agric. Food Chem. 2010, 58, 4617-4625). In the case of acrolein (**VI**), this compound may be originated as result of a thermic degradation of allicin in aqueous medium (Scheme 2, (a)), the hydrolysis of cation **VIII** (Scheme 2, (B)), intermediate in the synthesis of ajoene (Ilić, D. et al. Sci. World J. 2012, 2012, 561823), or via thermic or photochemical degradation of cysteine (Obata, Y. et al. Agric. Biol. Chem. 1965, 29, 196-199):

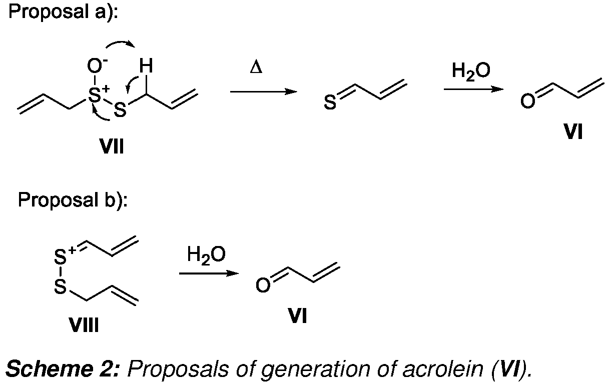

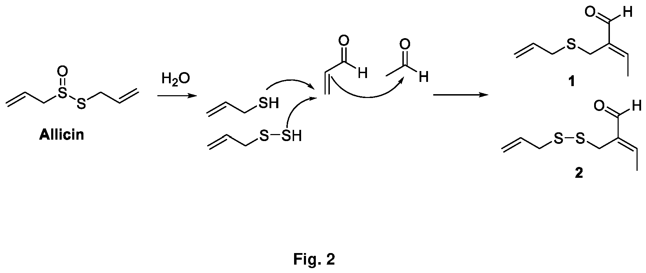

The reactions of formation of compounds **1** and **2** can take place in place in an aqueous medium where the approach of the organic synthons is favored. Under these circumstances a multicomponent reaction process as shown if Figure 2 is possible.

Once isolated, the activity of pure **1** and **2** were tested (Table 3).

**Table 3: Bioactivities of compound 1 and 2.**

| **Compound** | **Species** | **LD₅₀** (95% Confidence Limits) | **LD₉₀** (95% Confidence Limits) |
|---|---|---|---|
| **1** | *A. niger* | 78.07 (63.96-95.28)µg/mL | - |
| **1** | *B. cinerea* | 27.13 (21.09-27.12)µg/mL | - |
| **1** | *H. lusitanicum* | 8.42 (7.72-10.07) µg/mg | 15.62 (13.79-18.38) µg/mg |
| **2** | *A. niger* | 30.76 (24.39-38.78) µg/mL | - |
| **2** | *B. cinerea* | 10.10 (7.59-13.44) µg/mL | - |

The presence of a second sulfur atom in compound **1** increases up to three-four folds its activity with respect to that of compound **2.**

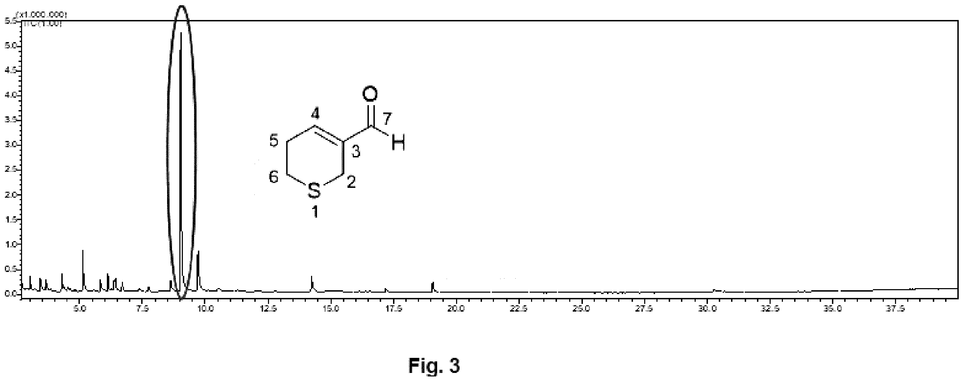

Fraction **D** was proven to possess a major compound after CG-MS analysis (Figure 3). This substance (**3**) was isolated via semi-preparative HPLC.

The HRMS of **3** showed a [M+H]⁺ at *m*/*z* 129.0374, which together with its ¹H and ¹³C NMR data (Table 4), established the molecular formula C₆H₉OS. This molecular formula, together with the analysis of its ¹³C NMR spectrum, permitted assign a cyclic structure for compound **3.**

**Table 4: NMR data of compound 3.**

| **Position** | **¹H (ppm)** | **¹³C (ppm)*** | **COSY** | **HMBC** |
|---|---|---|---|---|
| 1 | - | - | - | - |
| 2 | 3.33 *q* (*J* = 2.2 Hz) | 22.35 (CH₂) | H₃, H₄ | C₂, C₃ |
| 3 | - | 138.99 (C) | - | C₃ |
| 4 | 6.91 *m* | 150.93 (CH) | H₄ | - |
| 5 | 2.72 - 2.65 *m* | 27.53 (CH₂) | H₃, H₅ | C₃ |
| 6 | 2.80 *t* (*J* = 5.7 Hz) | 24.72 (CH₂) | H₄ | C₁, C₃, C₄ |
| 7 | 9.38 *s* | 192.78 (CH) | - | C₁, C₂ |

| | | | | |
|---|---|---|---|---|
| **HSOC-based assignment.* | | | | |

The ¹H NMR signals at δ 9.38 ppm δ 6.91 ppm suggested the existence of an α,β-unsaturated aldehyde in the structure of **3.** Signals corresponding to three methylene groups complete the ¹H NMR spectrum. Two of these methylene groups were assigned to be bound to the sulfur atom (H6, 2.80 *t* (*J* = 5.7 Hz); H2, 3.33 *q* (*J* = 2.2 Hz)), whereas the remaining one was located α to the olefinic proton as confirmed after analysis of the correlations observed in its 2D COSY and HMBC spectra (Table 1). Compound **3** was thus assigned the structure of 5,6-dihydro-2*H*-thiopyran-3-carbaldehyde.

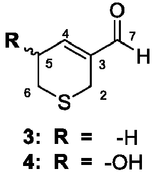

Although this compound was reported to be synthesized McIntosh and Khalil (McIntosh, J.M. et al. J. Org. Chem. 1977, 42, 2123-2126), this is the first time that aldehyde **3** is isolated from natural sources. Both synthetic and natural **3** display identical spectroscopic properties.

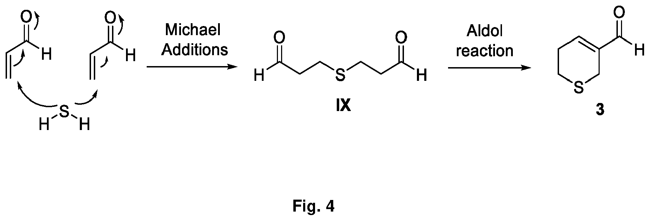

The generation of compound **3** can be rationalized as shown in Figure 4. Thus, **3** would be generated after an intramolecular aldolic reaction of intermediate **IX,** which, in turn, would be the result of two Michel additions between two molecules of acrolein (**VI**) and one molecule of hydrogen sulfide; the latter formed from the degradation of cysteine.

The activity of compound 3 was tested (Table 5).

**Table 5: Bioactivitie of compound 3.**

| **Compound** | **Species** | **LD₅₀** (95% Confidence Limits) | **LD₉₀** (95% Confidence Limits) |
|---|---|---|---|
| **3** | *A. niger* | 16.96 (7.98-36.03) µg/mL | - |
| **3** | *B. cinerea* | 8.79 (5.36-14.43) µg/mL | - |
| **3** | *H. lusitanicum* | - | 3.57 (3.20-4.23) µg/mg |

These compounds (**1-3**) are small molecules (<10 carbons) that contain an aldehyde group. This aldehyde can be hydrated to generate a gem-diol group, that should be soluble in hot water and retain the compounds in the hydrolate.

## Claims

1. Use of a compound of formula 1, 2 or 3, 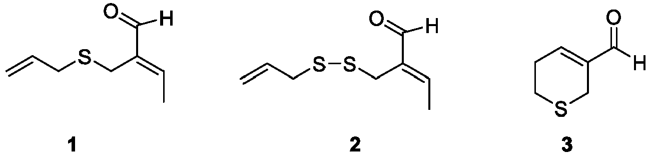 or a composition comprising the compound of formula 1, 2, 3 or any combination thereof, as a biocide to control phytopathogens or pest organisms that affect plants.

2. Use, according to claim 1, wherein the composition also comprises vehicles or agents selected from surfactants, dispersants and stabilizers to facilitate its conservation, handling and application.

3. Use, according to any of claim 1 or 2, wherein the phytopathogens or pest organisms are selected from fungi and ticks.

4. Use, according to claim 3, wherein the fungi are selected from *Aspergillus niger* and *Botrytis cinerea* and/or the species of ticks is *Hyalomma lusitanicum.*

5. Method to control phytopathogens or pest organisms that affect plants, wherein said method comprises treating the plant with an effective amount of a compound of formula 1, 2, or 3 or a composition comprising an effective amount of the compound of formula 1, 2, 3 or any combination thereof, as defined in any one of claim 1 or 2.

6. Method, according to claim 5, wherein the phytopathogens or pest organisms are fungi selected from *Aspergillus niger and Botrytis cinerea* and/or the species of ticks is *Hyalomma lusitanicum.*

7. Method, according to claim 5 or 6, wherein the compound or the composition is applied by its direct spraying onto the plant where the organism is found.

8. Compound of formula 1, 2 or 3: 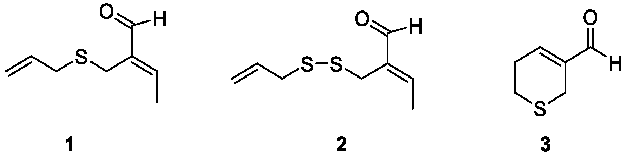 or a composition comprising the compound of formula 1, 2, 3 or any combination thereof, for use to treat an animal infested with an ectoparasite.

9. Compound or a composition for use according to claim 8, wherein the ectoparasite is selected form fungi and ticks.

10. Compound or a composition for use according to claim 9, wherein the fungi are selected from A*spergillus niger* and *Botrytis cinerea* and the tick species is *Hyalomma lusitanicum.*

11. Compound, or a composition, for use according to any of claims 8 to 10, wherein the compound or the composition is administered topically, preferably is applied by direct spraying onto the animal where the ectoparasite is located.

12. Biocidal composition comprising a compound of formula 1, 2, 3: 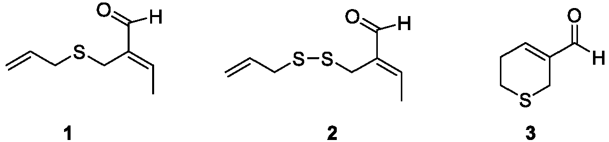 or any combination thereof.

13. Biocidal composition, according to claim 12, additionally comprising vehicles or agents selected from surfactants, dispersants and stabilizers to facilitate its conservation, handling and application.

14. Compound of formula 1 or 2:
